# EUROPEAN PATENT APPLICATION

(11) **EP 3 106 080 A1**
(43) Date of publication of application: **21.12.2016**
(21) Application number: 15749219.0
(22) Date of filing: 05.02.2015
(51) Int. Cl.: A61B 1/04, A61B 1/00, G02B 23/24

(54) **ENDOSCOPE SYSTEM**

(30) Priority: 14.02.2014 JP 2014026834
(71) Applicant: Olympus Corporation, Tokyo 192-8507 (JP)
(72) Inventor: HONDA Kazuki, Tokyo 151-0072 (JP); TAKAHASHI Takeshi, Hachioji-shi, Tokyo 192-8507 (JP); KURA Yasuhito, Hachioji-shi, Tokyo 192-8507 (JP)
(74) Representative: Gunzelmann, Rainer
(86) International application number: PCT/JP2015/053276
(87) International publication number: WO 2015/122355

(57) **Abstract**

An endoscope system 1 includes an insertion portion 4 inserted into an object, a direct-view observation window 11a provided in the insertion portion 4 and configured to acquire a first object image from a first region of the object, a side-view observation window 11b provided in the insertion portion 4 and configured to acquire a second object image from a second region of the object which is at least partially different from the first region, and a video processor 32 configured to generate an image signal generating a second image signal from the second object image by performing image processing as well as generating a first image signal from the first object image so that a width of an image region increases as a distance from a region adjacent to the first image signal increases in a positional relationship with the first image signal.

## Description

### Technical Field

The present invention relates to an endoscope system, and more particularly, to an endoscope system capable of observing a direct-view direction and a side-view direction simultaneously.

### Background Art

Endoscope systems provided with an endoscope that picks up an image of an object inside a subject and an image processing apparatus that generates an observation image of the object whose image is picked up by the endoscope are widely used in a medical field, an industrial field and the like.

For example, Japanese Patent Publication No. 3337682 discloses an endoscope system provided with an endoscope including a direct-view observation lens configured to acquire a direct-view visual field image provided on a distal end surface of a distal end portion of an insertion portion and a plurality of side-view observation lenses configured to acquire side-view visual field images provided in a circumferential direction of the distal end portion.

This endoscope is provided with image pickup devices at image forming positions of the direct-view observation lens and the plurality of side-view observation lenses respectively and a direct-view visual field image and a plurality of side-view visual field images are picked up by the respective image pickup devices. The direct-view visual field image is arranged at a center and the plurality of side-view visual field images are arranged on both sides of the direct-view visual field image and displayed on a monitor.

However, it is difficult to obtain perspective and three-dimensional effects using a conventional display scheme that simply arranges object images (endoscopic images) obtained from a plurality of image pickup sections and displays the object images on the monitor, and so it may be difficult to confirm which luminal area is being observed or predict a movement thereof. Furthermore, an endoscopic image without perspective may generate an unnatural feeling as a visual field. For this reason, there is a problem that it takes time and trouble to control the insertion or removal of the endoscope.

Thus, it is an object of the present invention to provide an endoscope system capable of improving viewability when an endoscope is inserted into a lumen and improving operability of the endoscope.

### Disclosure of Invention

### Means for Solving the Problems

An endoscope system according to an aspect of the present invention is provided with an insertion portion configured to be inserted into an object, a first object image acquisition section provided in the insertion portion and configured to acquire a first object image from a first region of the object, a second object image acquisition section provided in the insertion portion and configured to acquire a second object image from a second region of the object which is at least partially different from the first region, and an image signal generation section configured to generate an image signal when generating a second image signal from the second object image by performing image processing as well as generating a first image signal from the first object image so that a width of an image region increases as a distance from a region adjacent to the first image signal increases in a positional relationship with the first image signal.

### Brief Description of the Drawings

Fig. 1 is a diagram illustrating a configuration of an endoscope system according to a first embodiment;
Fig. 2 is a perspective view illustrating a configuration of a distal end portion of an insertion portion of an endoscope;
Fig. 3 is a diagram illustrating a configuration of main parts according to the first embodiment;
Fig. 4A is a diagram illustrating an example of an observation image displayed on a monitor through image processing by an image processing section 32a;
Fig. 4B is a diagram illustrating an example of an observation image displayed on the monitor through image processing by the image processing section 32a;
Fig. 5A is a diagram for describing specific shapes of side-view visual field images 17b to 17e;
Fig. 5B is a diagram for describing specific shapes of the side-view visual field images 17b to 17e;
Fig. 6 is a perspective view illustrating a configuration of a distal end portion of an insertion portion of an endoscope according to a second embodiment;
Fig. 7 is a diagram illustrating a configuration of main parts according to the second embodiment;
Fig. 8A is a diagram illustrating an example of an observation image displayed on a monitor through image processing by an image processing section 32a1;
Fig. 8B is a diagram illustrating an example of an observation image displayed on the monitor through image processing by the image processing section 32a1;
Fig. 8C is a diagram illustrating an example of an observation image displayed on the monitor through image processing by the image processing section 32a1;
Fig. 9 is a perspective view illustrating a configuration of a distal end portion of an insertion portion of an endoscope according to a modification;
Fig. 10 is a front view illustrating the configuration of the distal end portion of the insertion portion of the endoscope according to the modification;
Fig. 11 is a diagram illustrating a configuration of main parts according to the modification;
Fig. 12 is a diagram illustrating an example of an observation image displayed on a monitor through image processing by the image processing section 32a1;
Fig. 13 is a diagram illustrating a configuration of main parts according to a third embodiment;
Fig. 14 is a diagram illustrating an example of an observation image displayed on the monitor through image processing by an image processing section 32a2; and
Fig. 15 is a perspective view of the distal end portion 6 of the insertion portion 4 to which a side observation unit is attached.

### Best Mode for Carrying Out the Invention

Hereinafter, embodiments of the present invention will be described with reference to the accompanying drawings.

### (First Embodiment)

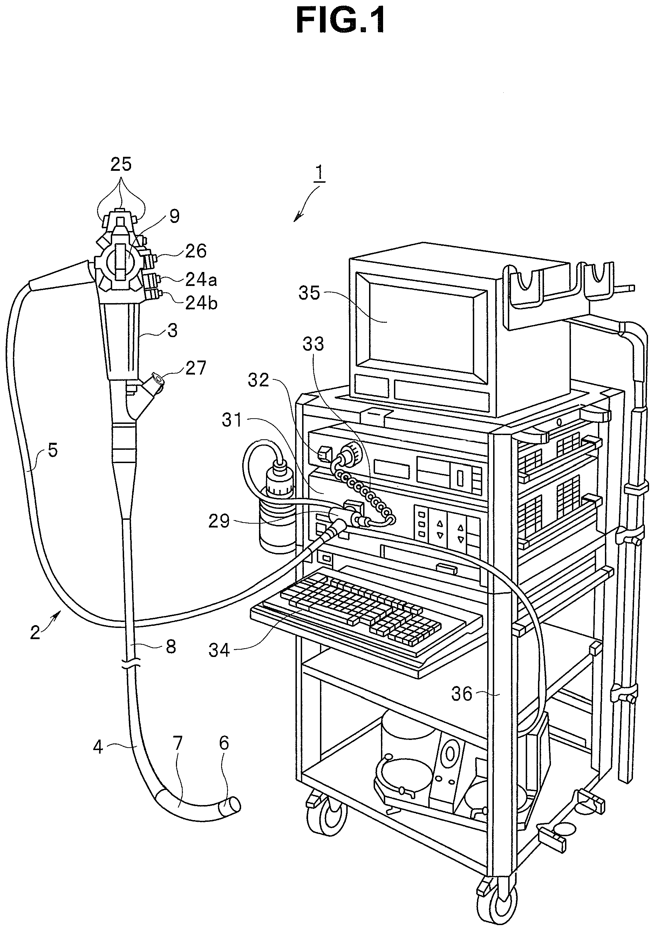

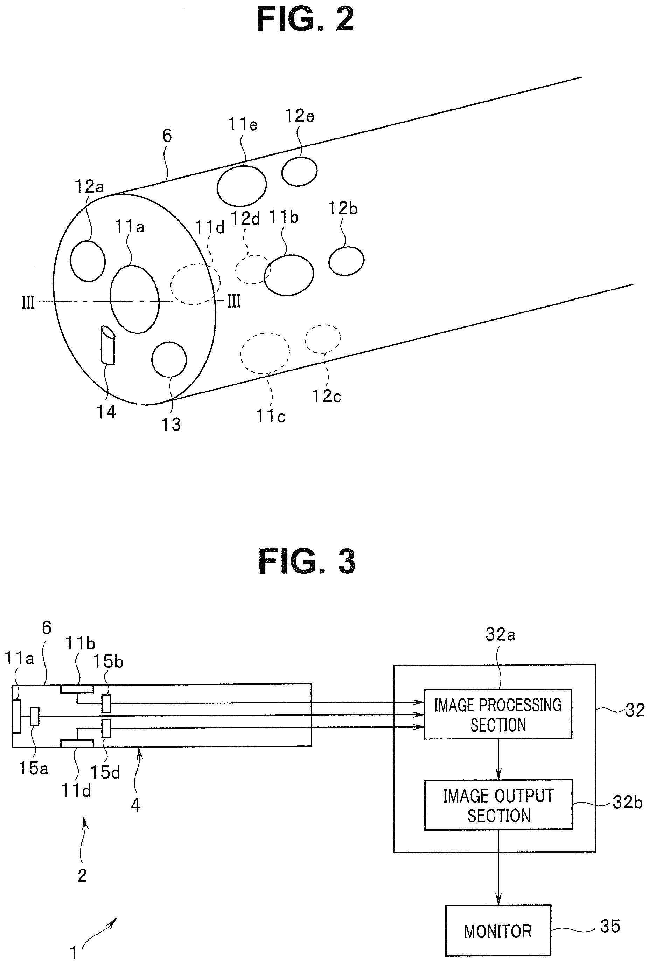

First, a configuration of an endoscope system according to a first embodiment will be described using Fig. 1 to Fig. 3. Fig. 1 is a diagram illustrating a configuration of the endoscope system according to the first embodiment, Fig. 2 is a perspective view illustrating a configuration of a distal end portion of an insertion portion of an endoscope and Fig. 3 is a diagram illustrating a configuration of main parts according to the first embodiment.

As shown in Fig. 1, an endoscope system 1 includes an endoscope 2 configured to pick up an image of an observation object and output an image pickup signal, a light source apparatus 31 configured to supply illuminating light to illuminate the observation object, a video processor 32 configured to possess a function as an image signal generation section that generates and outputs a video signal (image signal) corresponding to the image pickup signal, and a monitor 35 configured to display an observation image corresponding to the video signal (image signal).

The endoscope 2 is constructed of an operation portion 3 configured to be grasped by an operator to perform operation, an elongated insertion portion 4 formed on a distal end side of the operation portion 3 and inserted into a body cavity or the like, and a universal cord 5, one end of which is provided so as to extend from a side part of the operation portion 3.

The endoscope 2 according to the present embodiment is a wide-angle endoscope configured to display a plurality of visual field images and be capable of observing a visual field of 180 degrees or more and preventing overlooking of a lesion in a place difficult to detect only through observation in a direct-view direction such as the back of folds and a boundary of organs inside the body cavity, an interior of the large intestine in particular. When the insertion portion 4 of the endoscope 2 is inserted into the large intestine, operation such as temporary fixing by twisting, reciprocal motion of the insertion portion 2 and hooking of the intestinal wall is generated as in the case of a normal large intestine endoscope.

The insertion portion 4 is constructed of a rigid distal end portion 6 provided closest to the distal end side, a freely bendable bending portion 7 provided at a rear end of the distal end portion 6, and a long and flexible tube portion 8 provided at a rear end of the bending portion 7. Furthermore, the bending portion 7 performs bending operation corresponding to operation of a bending operation lever 9 provided at the operation portion 3.

On the other hand, as shown in Fig. 2, a direct-view observation window 11a configured to observe a direct-view direction (first direction) including a forward direction substantially parallel to a longitudinal direction of the insertion portion 4, that is, a first region of an object, is disposed on a distal end surface of the distal end portion 6 of the endoscope 2, and a plurality of side-view observation windows 11b, 11c, 11d and 11e configured to observe a side-view direction (second direction) including a direction crossing the longitudinal direction of the insertion portion 4 which is at least partially different from the direct-view direction (first direction), that is, a second region of the object are disposed on a side face of the distal end portion 6 of the endoscope 2. These side-view observation windows 11b to 11e are arranged in a circumferential direction of the distal end portion 6 at a uniform interval, for example, an interval of 90 degrees. Note that the side-view observation windows 11b to 11e arranged in the circumferential direction of the distal end portion 6 at a uniform interval are not limited to the four side-view observation windows, but a configuration may be adopted in which one or more side-view observation windows are arranged as a left and right pair (two), for example.

A direct-view illuminating window 12a configured to emit illuminating light over a range of the direct-view visual field of the direct-view observation window 11a is disposed on the distal end surface of the distal end portion 6 of the endoscope 2 at a position adjacent to the direct-view observation window 11a. Furthermore, side-view illuminating windows 12b to 12e configured to emit illuminating light over ranges of the side-view visual fields of the side-view observation windows 11b to 11e are arranged on a side face of the distal end portion 6 of the endoscope 2 at positions adjacent to the side-view observation windows 11b to 11e respectively.

A distal end opening 13 configured to communicate with a treatment instrument channel, which is not shown, formed of a tube or the like and disposed in the insertion portion 4 and cause (a distal end portion of) a treatment instrument inserted through the treatment instrument channel to protrude, and a direct-view observation window nozzle portion 14 configured to eject a gas or liquid to clean the direct-view observation window 11a are provided on the distal end surface of the distal end portion 6 of the endoscope 2. Furthermore, side-view observation window nozzle portions, which are not shown, configured to eject a gas or liquid to clean the side-view observation windows 11b to 11e are provided on the side face of the distal end portion 6 of the endoscope 2 adjacent to the side-view observation windows 11b to 11e respectively.

A gas/liquid feeding operation button 24a that can instruct operation of ejecting a gas or liquid to clean the direct-view observation window 11a from the direct-view observation window nozzle portion 14 and a gas/liquid feeding operation button 24b that can instruct operation of ejecting a gas or liquid to clean the side-view observation windows 11 b to 11e from a side-view observation window nozzle portion, which is not shown, are provided at the operation portion 3 as shown in Fig. 2, and it is possible to switch between gas feeding and liquid feeding by pressing the gas/liquid feeding operation buttons 24a and 24b. In the present embodiment, a plurality of gas/liquid feeding operation buttons are provided so as to correspond to the respective nozzle portions, but the present embodiment may be configured so that a gas or liquid is ejected from both the direct-view observation window nozzle portion 14 and the side-view observation window nozzle portions which are not shown, for example, through operation of one gas/liquid feeding operation button.

A plurality of scope switches 25 are provided at a top of the operation portion 3 and configured such that functions specific to the respective switches can be assigned thereto so as to output signals corresponding to ON, OFF or the like of various descriptions available to the endoscope 2. More specifically, the scope switches 25 can be assigned functions of outputting signals corresponding to the starting and stopping of forward water feeding, execution and releasing of freezing, announcement of an operating condition of a treatment instrument or the like as functions specific to the respective switches.

Note that at least one of the functions of the gas/liquid feeding operation buttons 24a and 24b may be assigned to one of the scope switches 25 in the present embodiment.

Furthermore, a suction operation button 26 configured to be able to instruct a suction unit or the like, which is not shown, to suction and collect mucus or the like in the body cavity from the distal end opening 13 is disposed at the operation portion 3.

The mucus or the like inside the body cavity suctioned in response to the operation of the suction unit or the like, which is not shown, is passed through the distal end opening 13, the treatment instrument channel, which is not shown, in the insertion portion 4 and a treatment instrument insertion opening 27 provided in the vicinity of a front end of the operation portion 3, and then collected into a suction bottle or the like of the suction unit, which is not shown.

The treatment instrument insertion opening 27 communicates with the treatment instrument channel, which is not shown, in the insertion portion 4 and is formed as an opening through which a treatment instrument, which is not shown, can be inserted. That is, the operator inserts the treatment instrument from the treatment instrument insertion opening 27, causes the distal end side of the treatment instrument to protrude from the distal end opening 13, and can thereby perform treatment using the treatment instrument.

On the other hand, as shown in Fig. 1, a connector 29 which is connectable to the light source apparatus 31 is provided at the other end of the universal cord 5.

A pipe sleeve (not shown) which is a connection end of a fluid conduit and a light guide pipe sleeve (not shown) which is a supply end of illuminating light are provided at a distal end portion of the connector 29. Furthermore, an electric contact point (not shown) to which one end of a connection cable 33 is connectable is provided on a side face of the connector 29. Moreover, a connector to electrically connect the endoscope 2 to the video processor 32 is provided at the other end of the connection cable 33.

A plurality of signal lines to transmit various electric signals and a light guide to transmit illuminating light supplied from the light source apparatus 31 are bundled and incorporated in the universal cord 5.

The light guide incorporated from the insertion portion 4 to the universal cord 5 is configured such that an end thereof on the light-emitting side is branched into at least five directions in the vicinity of the insertion portion 4 and the respective light-emitting end faces are arranged at the direct-view illuminating window 12a and the side-view illuminating windows 12b to 12e. The light guide is configured such that an end thereof on the light incidence side is disposed at the light guide pipe sleeve of the connector 29.

Note that the light-emitting portions arranged at the direct-view illuminating window 12a and the side-view illuminating windows 12b to 12e may be light-emitting devices such as light-emitting diodes (LEDs) instead of light guides.

The video processor 32 outputs drive signals to drive a plurality of image pickup devices provided at the distal end portion 6 of the endoscope 2. The video processor 32 functions as an image signal generation section configured to apply signal processing to image pickup signals outputted from the plurality of image pickup devices, generate video signals (image signals) and output the video signals to the monitor 35.

Although details will be described later, the processor 32 arranges the direct-view visual field image acquired by the direct-view observation window 11a at the center and arranges the four side-view visual field images acquired by the side-view observation windows 11b to 11e above and below, and to the left and right of the direct-view visual field image, applies predetermined image processing (deformation processing) to the direct-view visual field image and the four side-view visual field images and outputs the images to the monitor 35. That is, the processor 32 performs treatment so as to arrange the direct-view visual field image acquired by the direct-view observation window 11a and the side-view visual field images acquired by the side-view observation windows 11b to 11e at positions adjacent to each other and generates a video signal.

Peripheral apparatuses such as the light source apparatus 31, the video processor 32 and the monitor 35 are arranged on a rack 36 together with a keyboard 34 configured to input patient information or the like.

As shown in Fig. 3, the direct-view observation window 11a that constitutes a first object image acquisition section acquires a first object image from a direct-view direction (first direction) including a forward direction substantially parallel to the longitudinal direction of the insertion portion 4, that is, from the first region of the object. An image pickup device 15a is disposed at an image forming position of the direct-view observation window 11a and an objective optical system, which is not shown, configured to photoelectrically convert the object image acquired by the direct-view observation window 11 a. Note that the insertion portion 4 shown in Fig. 3 is a cross-sectional view along a line III-III in Fig. 2.

On the other hand, the side-view observation windows that constitute a second object image acquisition section (at least one or more side-view observation windows of the side-view observation windows 11b to 11e) acquire second object images from a side-view direction (second direction) including a direction crossing the longitudinal direction of the insertion portion 4 which is at least partially different from the direct-view direction (first direction), that is, the second region of the object.

Note that boundary regions between the first object image and the second object images may overlap or may not overlap with each other, and when the above-described boundary regions overlap, the first object image acquisition section and the second object image acquisition section may acquire overlapping object images.

An image pickup device 15b is disposed at an image forming position of the side-view observation window 11b and an objective optical system, which is not shown, configured to photoelectrically convert an object image acquired by the side-view observation window 11b.

Similarly, an image pickup device 15d is disposed at an image forming position of the side-view observation window 11d and an objective optical system, which is not shown, configured to photoelectrically convert an object image acquired by the side-view observation window 11d. Note that an image pickup device, which is not shown, (hereinafter referred to as "image pickup device 15c") is disposed at an image forming position of the side-view observation window 11c and the objective optical system which is not shown and an image pickup device, which is not shown, (hereinafter referred to as "image pickup device 15e") is disposed at an image forming position of the side-view observation window 11e and an objective optical system, which is not shown. The object images acquired by the image pickup devices 15c and 15e through the side-view observation windows 11c and 11e are photoelectrically converted.

The image pickup devices 15a to 15e are respectively electrically connected to an image processing section 32a and the direct-view visual field image picked up by the image pickup device 15a and the side-view visual field images respectively picked up by the image pickup devices 15b to 15e are outputted to the image processing section 32a.

The image processing section 32a arranges the direct-view visual field image acquired by the direct-view observation window 11a at the center, arranges the four side-view visual field images acquired by the side-view observation windows 11b to 11e above and below, and to the left and right of the direct-view visual field image, applies predetermined image processing to the direct-view visual field image and the four side-view visual field images and outputs the images to an image output section 32b.

The image output section 32b generates a signal to be displayed on the monitor 35 from the image signal generated by the image processing section 32a and outputs the signal to the monitor 35.

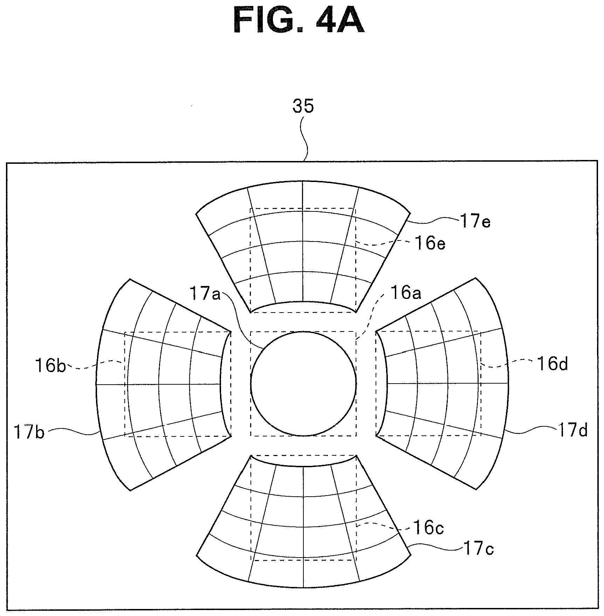

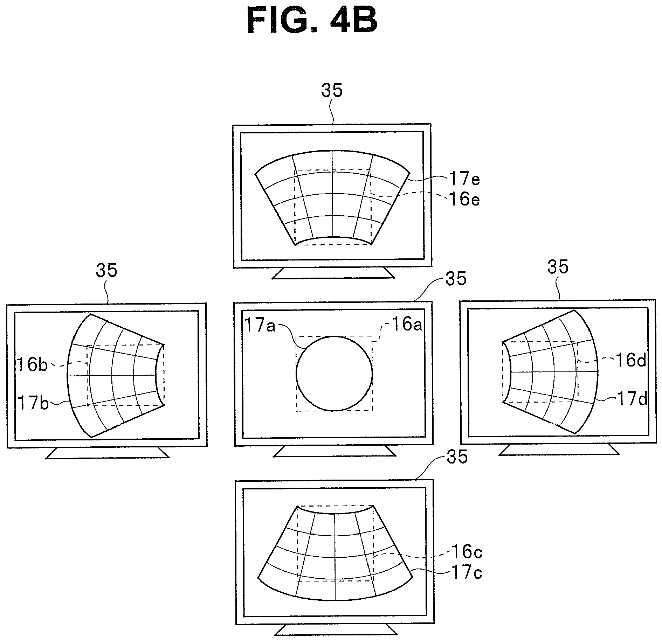

Next, image processing by the image processing section 32a will be described using Fig. 4A and Fig. 4B.

Fig. 4A and Fig. 4B are diagrams illustrating an example of an observation image displayed on the monitor through the image processing by the image processing section 32a.

The image processing section 32a acquires the direct-view visual field image 16a acquired by the direct-view observation window 11a and the side-view visual field images 16b to 16e acquired by the side-view observation windows 11b to 11e. The image processing section 32a arranges the direct-view visual field image 16a at the center and arranges the side-view visual field images 16b to 16e adjacent to the direct-view visual field image 16a in the vertical and horizontal directions as shown in Fig. 4A. More specifically, the image processing section 32a arranges the side-view visual field image 16b on the left side of the direct-view visual field image 16a, arranges the side-view visual field image 16c below the direct-view visual field image 16a, arranges the side-view visual field image 16d on the right side of the direct-view visual field image 16a and arranges the side-view visual field image 16e above the direct-view visual field image 16a.

The image processing section 32a then applies predetermined image processing to the direct-view visual field image 16a and the side-view visual field images 16b to 16b. More specifically, the image processing section 32a applies circular electronic masking to the direct-view visual field image 16a and generates a substantially circular direct-view visual field image 17a.

Furthermore, the image processing section 32a applies deformation processing (distortion) to the side-view visual field images 16b to 16e arranged above and below, and to the left and right so that the images are expanded as their distances from the center increase, that is, the widths of the image regions increase as their distances from the regions adjacent to the direct-view visual field image 16a increase in a positional relationship with the direct-view visual field image 16a and generates substantially fan-shaped side-view visual field images 17b to 17e. The substantially circular direct-view visual field image 17a and the fan-shaped side-view visual field images 17b to 17e generated by the image processing section 32a are displayed on the monitor 35 via the image output section 32b.

Thus, the image processing section 32a performs image processing in a width expansion mode which is an image processing mode in which the image processing section 32a generates a first image signal from a first object image, generates a second image signal from a second object image and processes the second image signal so that the width of the second object image increases as the distance from the center of the first object image increases.

Note that when a plurality of images are displayed on the monitor 35, the side-view visual field images 17b to 16e are configured to be arranged above and below, and to the left and right of the direct-view visual field image 17a, but without being limited to this, the direct-view visual field image and the side-view visual field images only need to neighbor each other, and a configuration may be adopted in which the side-view visual field image is disposed either to the left or right of the direct-view visual field image 17a.

In the present embodiment, a plurality of images are displayed on the monitor 35, but the present embodiment is not limited to this. As shown in Fig. 4B, such a configuration may be adopted in which a plurality of, for example, five monitors 35 are arranged adjacent to each other, the direct-view visual field image 17a is displayed on the central monitor 35 and the side-view visual field images 17b to 17e are displayed on the upper, lower, left and right monitors 35 respectively.

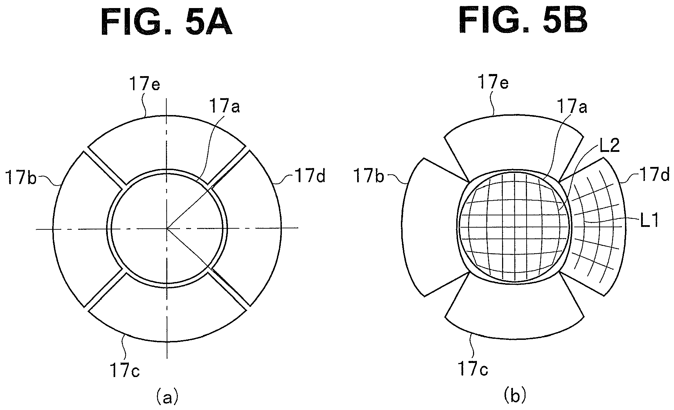

Note that specific shapes of the side-view visual field images 17b to 17e generated by the image processing section 32a may be as shown in Fig. 5A and Fig. 5B. Fig. 5A and Fig. 5B are diagrams for describing the specific shapes of the side-view visual field images 17b to 17e.

As shown in Fig. 5A, the image processing section 32a may generate fan-shaped side-view visual field images 17b to 17e concentric to the direct-view visual field image 17a and having an internal angle of substantially 90 degrees. Furthermore, the image processing section 32a may generate fan-shaped side-view visual field images 17b to 17e where a distortion (curvature of field) level L1 of the side-view visual field images 17b to 17e becomes substantially equal to a distortion level L2 of the peripheral part of the direct-view visual field image 17a as shown in Fig. 5B.

The direct-view visual field image 17a originally contains a certain degree of distortion in its peripheral part for reasons related to the optical system. For this reason, the image processing section 32a performs image processing so that the side-view visual field images 17b to 17e become distorted images in accordance with the peripheral part of the direct-view visual field image 17a (so as to obtain radial perspective). In that case, the image processing section 32a may set slightly wider visual fields for the side-view visual field images 17b to 17e so as to overlap the direct-view visual field image 17a extracted using electronic masking so as to complement the shortfall (electronically masked portion) of the direct-view visual field image 17a when images are superimposed one on another.

In this way, the endoscope system 1 acquires the direct-view visual field image 16a through the direct-view observation window 11a, acquires the side-view visual field images 16b to 16e through the side-view observation windows 11b to 11e, arranges the direct-view visual field image 16a at the center and arranges the side-view visual field images 16b to 16e above and below, and to the left and right of the direct-view visual field image 16a. The endoscope system 1 generates the substantially circular direct-view visual field image 17a resulting from applying circular electronical masking to the direct-view visual field image 16a and the fan-shaped side-view visual field images 17b to 17e resulting from applying deformation processing to the side-view visual field images 16b to 16e so that their widths increase as the respective distances from the regions adjacent to the direct-view visual field image 16a increase.

As a result, since the direct-view visual field image 17a and the side-view visual field images 17b to 17e displayed on the monitor 35 can produce perspective, it is possible to improve viewability in a cylindrical lumen such as the large intestine and improve operability.

Thus, according to the endoscope system of the present embodiment, it is possible to improve viewability and improve operability of the endoscope when the endoscope is inserted into the lumen.

### (Second Embodiment)

Next, a second embodiment will be described.

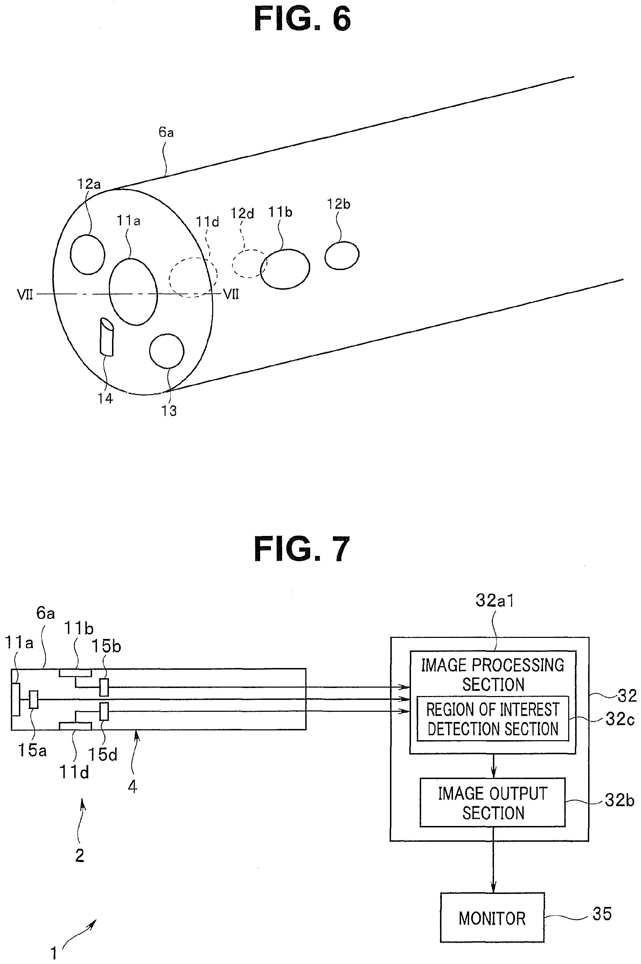

Fig. 6 is a perspective view illustrating a configuration of a distal end portion of an insertion portion of an endoscope according to a second embodiment and Fig. 7 is a diagram illustrating a configuration of main parts according to the second embodiment. Note that in Fig. 6 and Fig. 7, components similar to those in Fig. 2 and Fig. 3 are assigned the same reference numerals and description thereof will be omitted.

As shown in Fig. 6, a side face of the distal end portion 6a of the endoscope 2 according to the present embodiment is configured by removing the side-view observation windows 11c and 11e and the side-view illuminating windows 12c and 12e from the side face of the distal end portion 6 in Fig. 2. That is, the endoscope 2 of the present embodiment acquires the direct-view visual field image 16a through the direct-view observation window 11a and acquires the two side-view visual field images 16b and 16d through the side-view observation windows 11b and 11d.

As shown in Fig. 7, the video processor 32 according to the present embodiment is constructed using an image processing section 32a1 instead of the image processing section 32a in Fig. 3. The image processing section 32a1 arranges the direct-view visual field image 16a acquired by the direct-view observation window 11a at the center, arranges the side-view visual field images 16b and 16d acquired by the side-view observation windows 11b and 11d side by side to the left and right of the direct-view visual field image 16a, and applies predetermined image processing (deformation processing) to the side-view visual field images 16b and 16d.

The image processing section 32a1 is provided with a region of interest detection section 32c. The region of interest detection section 32c detects a predetermined region of interest such as a lesion in the side-view visual field image subjected to deformation processing by detecting, for example, a color tone change. When a lesion is detected by the region of interest detection section 32c, the image processing section 32a1 cancels the deformation processing on the side-view visual field image subjected to the deformation processing in which the lesion is reflected, and displays an enlarged image thereof. Note that when there is any part whose color tone is different from other parts in the side-view visual field image (when a lesion is detected), that part may be extracted and an enlarged image thereof may be displayed.

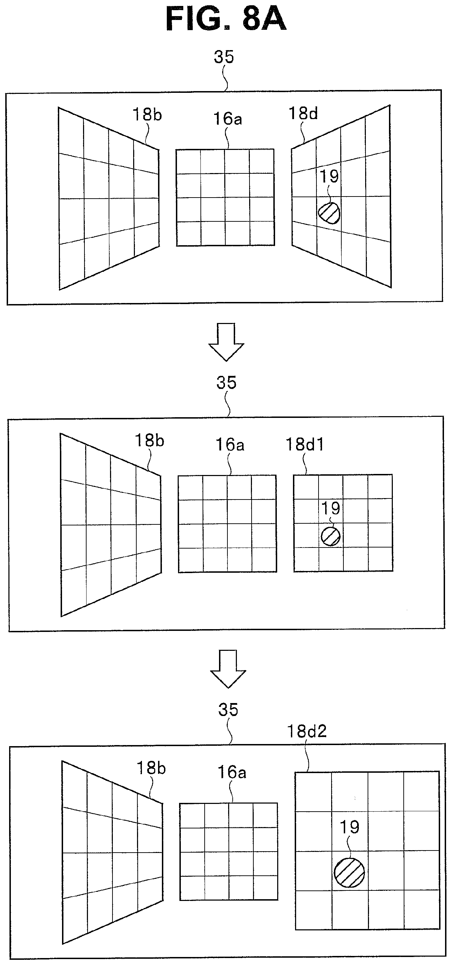

Next, image processing by the image processing section 32a1 will be described using Fig. 8A to Fig. 8C.

Fig. 8A to Fig. 8C are diagrams illustrating an example of an observation image displayed on a monitor through image processing by the image processing section 32a1.

The image processing section 32a1 acquires the direct-view visual field image 16a acquired by the direct-view observation window 11a and the side-view visual field images 16b and 16d acquired by the side-view observation windows 11b and 11d. The image processing section 32a1 arranges the direct-view visual field image 16a at the center and arranges the side-view visual field images 16b and 16d side by side to the left and right of the direct-view visual field image 16a.

The image processing section 32a1 applies deformation processing to the side-view visual field images 16b and 16d so that the side-view visual field images 16b and 16d are expanded as their distances from the respective regions adjacent to the direct-view visual field image 16 increase to thereby generate side-view visual field images 18b and 18d respectively. More specifically, trapezoidal side-view visual field images 18b and 18d are generated, whose lengths of sides close to the direct-view visual field image 16a are substantially identical to the length of the side of the direct-view visual field image 16a and whose lengths of sides far from the direct-view visual field image 16a are longer than the length of the sides closer to the direct-view visual field image 16a. The direct-view visual field image 16a, and the side-view visual field images 18b and 18d subjected to the deformation processing are displayed on the monitor 35 via the image output section 32b.

When the region of interest detection section 32c detects a lesion 19 in, for example, the side-view visual field image 18d, the image processing section 32a1 causes the monitor 35 to display a side-view visual field image 18d1 resulting from cancelling the deformation processing of the side-view visual field image 18d in which the lesion is reflected. Furthermore, the image processing section 32a1 generates a side-view visual field image 18d2 which is an enlarged image of the side-view visual field image 18d1 whose deformation processing is canceled and causes the monitor 35 to display this side-view visual field image 18d2.

Thus, the endoscope system 1 generates the side-view visual field images 18b and 18d subjected to deformation processing whereby the side-view visual field images 18b and 18d are expanded as their distances from the center increase, and can thereby improve viewability and improve operability of the endoscope when the endoscope is inserted into the lumen as in the case of the first embodiment. Upon detecting the lesion 19 in the side-view visual field image 18b or 18d, the endoscope system 1 cancels the deformation processing on the side-view visual field image 18b or 18d in which the lesion 19 is detected, and enable thereby observation of the lesion 19 in an image free of distortion.

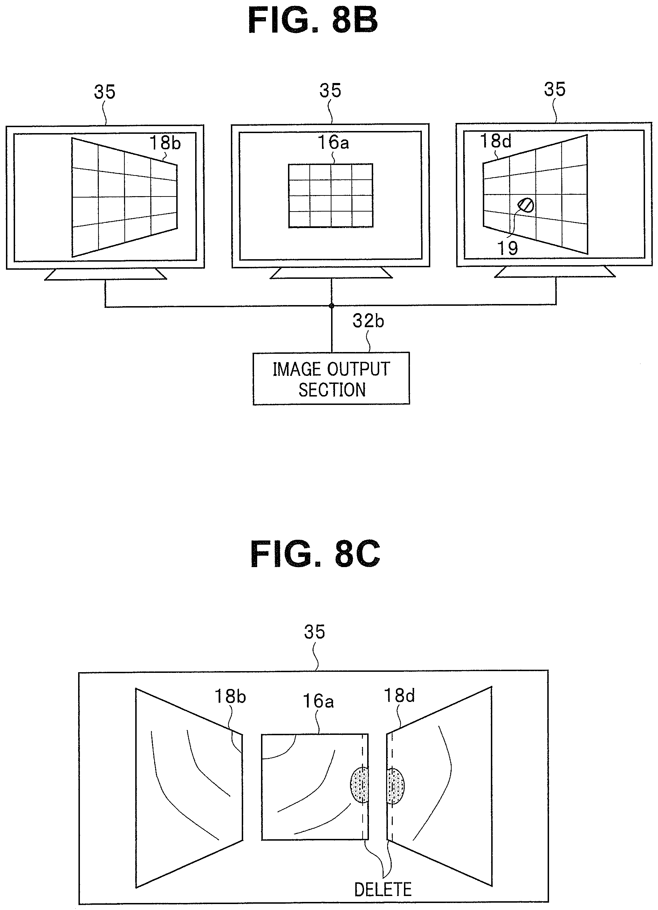

Note that as shown in Fig. 8B, the direct-view visual field image 16a and the side-view visual field images 16b and 16d (18b and 18d) may be displayed on the monitor 35 in plurality respectively. Alternatively, as shown in Fig. 8C, if there is an overlapping part between two neighboring image signals, the images may be displayed by deleting the overlapping part from the image signals.

### (Modification)

Next, a modification of the second embodiment will be described.

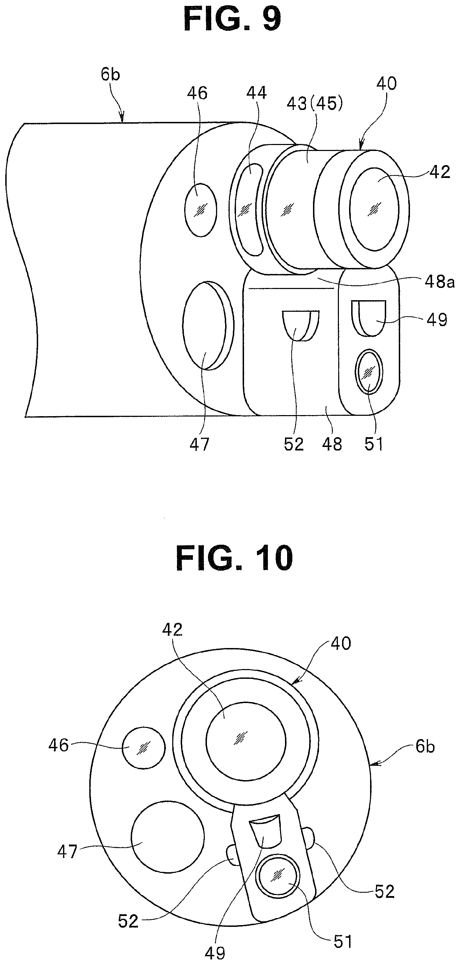

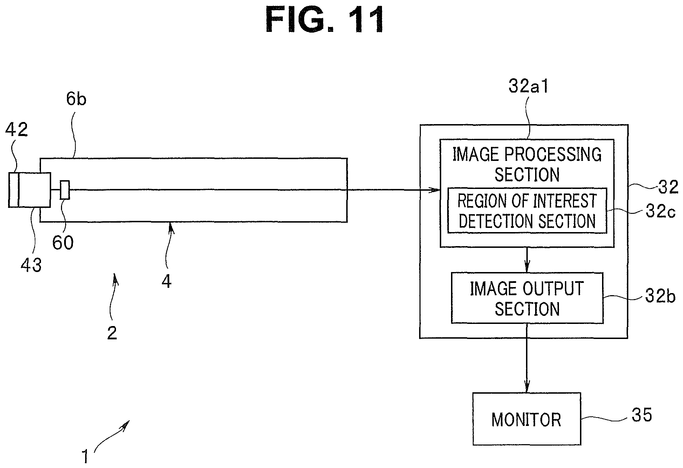

Fig. 9 is a perspective view illustrating a configuration of a distal end portion of an insertion portion of an endoscope according to a modification, Fig. 10 is a front view illustrating a configuration of the distal end portion of the insertion portion of the endoscope according to the modification and Fig. 11 is a diagram illustrating a configuration of main parts according to the modification.

As shown in Fig. 9, a columnar cylindrical portion 40 is formed at the distal end portion 6b of the insertion portion 4, protruding from a position deviated upward from the center of the distal end surface of the distal end portion 6b.

An objective optical system, which is not shown and configured to provide both a direct-view and a side-view, is provided at a distal end portion of the cylindrical portion 40. The distal end portion of the cylindrical portion 40 is configured to include a direct-view observation window 42 that constitutes a first object image acquisition section disposed at a position corresponding to a direct-view direction of the objective optical system which is not shown, and a side-view observation window 43 that constitutes a second object image acquisition section disposed at a position corresponding to a side-view direction of the objective optical system, which is not shown. Furthermore, a side-view illumination section 44 configured to emit light for illuminating the side-view direction is formed in the vicinity of a proximal end of the cylindrical portion 40.

The direct-view observation window 42 captures return light (reflected light) from an observation object incident from a first region including a forward direction of the insertion portion 4 substantially parallel to the longitudinal direction of the insertion portion 4 within the direct-view visual field as a direct-view object image, and thereby acquires a direct-view visual field image.

The side-view observation window 43 captures return light (reflected light) from the observation object incident from a circumferential direction of the columnar cylindrical portion 40 within the side-view visual field, and is provided with a side-view mirror lens 45 to thereby allow a side-view visual field image to be acquired.

Such an image is realized using a two-time reflection optical system in which the return light is reflected twice by the side-view mirror lens 45, but such an image may be formed by causing the return light to be reflected once by a one-time reflection optical system, subjecting the image to image processing by the video processor 32 and matching the orientation of the side-view visual field image to that of the direct-view visual field image.

Note that (an image pickup surface of) an image pickup device 60 shown in Fig. 11 is assumed to be disposed at an image forming position of the objective optical system, which is not shown, so that an image of an observation object within a visual field of the direct-view observation window 42 is formed at a central part as a circular direct-view visual field image and an image of an observation object within a visual field of the side-view observation window 43 is formed on an outer circumferential portion of the direct-view visual field image as a ring-shaped side-view visual field image.

A direct-view illuminating window 46 disposed at a position adjacent to the cylindrical portion 40 and configured to emit illuminating light within a range of the direct-view visual field of the direct-view observation window 42 and a distal end opening 47 configured to communicate with a treatment instrument channel, which is formed of a tube or the like disposed in the insertion portion 4 and which is not shown, and be enabled to cause (a distal end portion of) the treatment instrument inserted through a treatment instrument channel to protrude therefrom are provided on the distal end surface of the distal end portion 6b.

The distal end portion 6b of the insertion portion 4 includes a supporting portion 48 provided so as to protrude from the distal end surface of the distal end portion 6b and this supporting portion 48 is located below and adjacent to the cylindrical portion 40.

The supporting portion 48 is configured to be able to support (or hold) each protruding member disposed so as to protrude from the distal end surface of the distal end portion 6b. More specifically, the supporting portion 48 is configured to be able to support (or hold) a direct-view observation window nozzle portion 49 configured to eject a gas or liquid to clean the direct-view observation window 42, a direct-view illuminating window 51 configured to emit light for illuminating a direct-view direction, and a side-view observation window nozzle portion 52 configured to eject a gas or liquid to clean the side-view observation window 43, as each aforementioned protruding member.

On the other hand, the supporting portion 48 includes a shielding portion 48a which is an optical shielding member configured to prevent acquisition of a side-view visual field image that may include any one of the respective protruding members when each aforementioned protruding member which is an object different from original observation objects appears within the side-view visual field. That is, by providing the supporting portion 48 with the shielding portion 48a, it is possible to obtain a side-view visual field image that includes none of the direct-view observation window nozzle portion 49, the direct-view illuminating window 51 or the side-view observation window nozzle portion 52.

As shown in Fig. 9 and Fig. 10, the side-view observation window nozzle portion 52 is provided at two locations of the supporting portion 48 and is disposed such that the distal end thereof protrudes from the side face of the supporting portion 48.

The video processor 32 outputs a drive signal to drive the image pickup device 60 provided at the distal end portion 6b of the endoscope 2. The video processor 32 applies signal processing to an image pickup signal outputted from the image pickup device 60, thereby generates a video signal and outputs the video signal to the monitor 35. Thus, the monitor 35 displays an observation image including a circular direct-view visual field image and a ring-shaped side-view visual field image arranged adjacent to the direct-view visual field image and around an outer circumference of the direct-view direction image. Note that the portion optically shielded by the shielding portion 48a of the supporting portion 48 will not be considered in observation images shown in the present embodiment and subsequent embodiments.

It is not possible to obtain perspective or a three-dimensional effect only by arranging one or more side-view visual field images next to the direct-view visual field image and it is difficult to recognize the image obtained as an observation image of the luminal interior without any unnatural feeling.

In contrast, the method of displaying the direct-view visual field image and the side-view visual field images of the modification is set to provide an optical structure whereby the screen spreads radially from the center toward the periphery (such an optical characteristic is automatically set in the case of a ring-shaped lens), and perspective and a three-dimensional effect can therefore be obtained relatively easily.

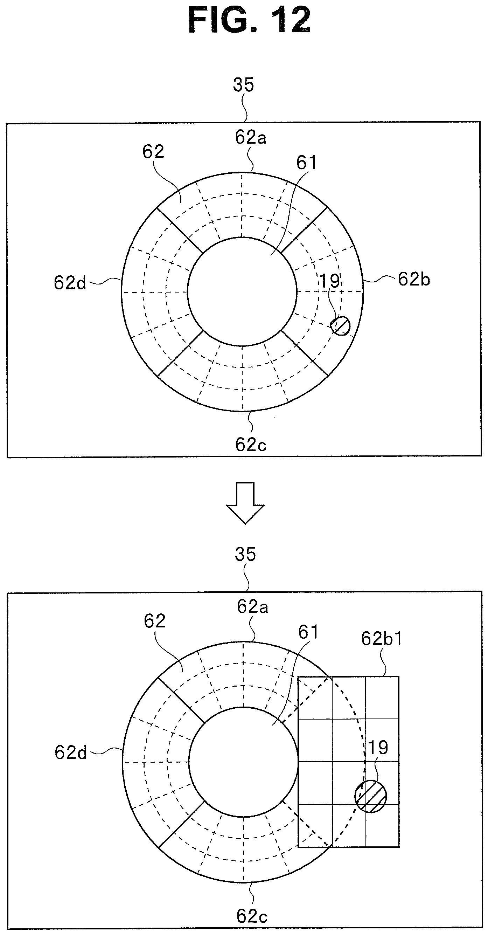

Next, image processing by the image processing section 32a1 will be described using Fig. 12.

Fig. 12 is a diagram illustrating an example of an observation image displayed on a monitor through image processing by the image processing section 32a1.

As shown in Fig. 12, the image processing section 32a1 acquires a circular direct-view visual field image 61 and a ring-shaped side-view visual field image 62 around an outer circumference of the direct-view visual field image 61. Moreover, the image processing section 32a1 divides the side-view visual field image 62 into four upper, lower, left and right regions 62a, 62b, 62c and 62d. Note that the number of divided regions is not limited to four, but may be three or less or five or more.

When the region of interest detection section 32c detects, for example, a lesion 19, the image processing section 32a1 applies only to, for example, the region 62b of the side-view visual field image 62 in which the lesion 19 is included, distortion elimination processing that cancels a state in which the width of the image region increases as the distance from the region adjacent to the direct-view visual field image increases, generates an enlarged image 62b1 and displays the enlarged image 62b1 on the monitor 35 as a switchover mode. As a result, the endoscope system 1 of the modification cancels the deformation processing on the region in which the lesion 19 is detected, and can thereby observe the lesion 19 in a distortion-free image as in the case of the second embodiment.

### (Third Embodiment)

Next, a third embodiment will be described.

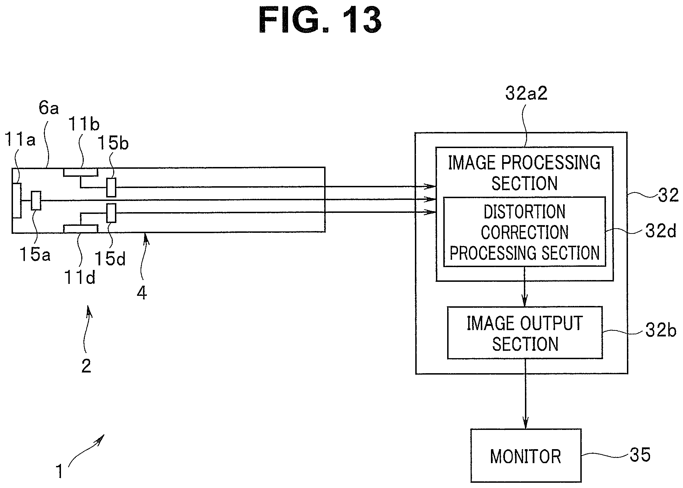

Fig. 13 is a diagram illustrating a configuration of main parts according to the third embodiment. Note that in Fig. 13, components similar to those in Fig. 7 are assigned the same reference numerals and description thereof will be omitted. The configuration of the distal end portion of the insertion portion 4 is similar to that of the distal end portion 6a in Fig. 6.

As shown in Fig. 13, the video processor 32 of the present embodiment is configured using an image processing section 32a2 instead of the image processing section 32a1 in Fig. 7. The image processing section 32a2 arranges the direct-view visual field image 16a acquired by the direct-view observation window 11a at the center, arranges the side-view visual field images 16b and 16d acquired by the side-view observation windows 11b and 11d side by side to the left and right of the direct-view visual field image 16a, and applies predetermined image processing (deformation processing) to the side-view visual field images 16b and 16d.

The image processing section 32a1 is provided with a distortion correction processing section 32d configured to correct distortion in horizontal and vertical directions. The distortion correction processing section 32d applies distortion elimination processing to the direct-view visual field image 16a and the two side-view visual field images subjected to the deformation processing so as to clear distortion to zero. The direct-view visual field image and the two side-view visual field images subjected to the distortion elimination processing are displayed on the monitor 35 via the image output section 32b.

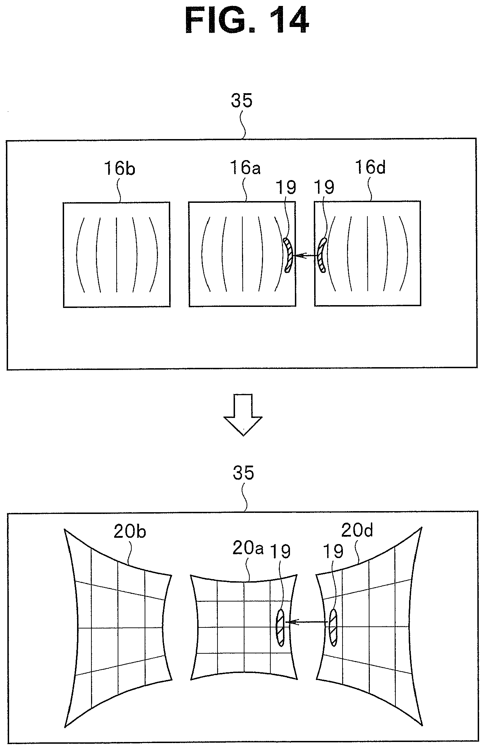

Next, the image processing by the image processing section 32a2 will be described using Fig. 14.

Fig. 14 is a diagram illustrating an example of an observation image displayed on the monitor through the image processing by the image processing section 32a2.

Distortion generally exists in the acquired direct-view visual field image 16a and side-view visual field images 16b and 16d, and the distortion direction differs in the vicinity of the boundary between the direct-view visual field image 16a and the side-view visual field images 16b and 16d. For this reason, when an object (e.g., lesion 19) moves between the respective images, even the identical object differs in its appearance and behavior, and it is therefore difficult to recognize that it is the identical object.

Thus, the distortion correction processing section 32d generates a direct-view visual field image 20a by applying to the direct-view visual field image 16a, distortion elimination processing that sets distortion to zero. Furthermore, the distortion correction processing section 32d generates side-view visual field images 20b and 20d by applying distortion elimination processing that sets distortion to zero to the side-view visual field images subjected to deformation processing by the image processing section 32a2 so that the side-view visual field images 16b and 16d are expanded as the distances from the center increase.

The direct-view visual field image 20a, and the side-view visual field images 20b and 20d subjected to the distortion elimination processing are displayed on the monitor 35 via an image output section 32b. Thus, for example, even when the object (lesion 19) of the side-view visual field image 20d moves to the direct-view visual field image 20a, its appearance and behavior become substantially identical. As a result, the endoscope system of the present embodiment exerts an effect of improving viewability when the object (lesion 19) moves from the side-view visual field image 20b or 20d to the direct-view visual field image 20a (or from the direct-view visual field image 20a to the side-view visual field image 20b or 20d) in addition to the effect of the first embodiment.

Of the above-described embodiments, according to the embodiments in which a plurality of visual field images are arranged side by side and displayed, the mechanism for implementing the function of illuminating and observing the lateral direction is incorporated in the distal end portion 6 of the insertion portion 4 together with the mechanism for implementing the function of illuminating and observing the forward direction, but the mechanism for implementing the function of illuminating and observing the lateral direction may be a separate body detachable from the insertion portion 4.

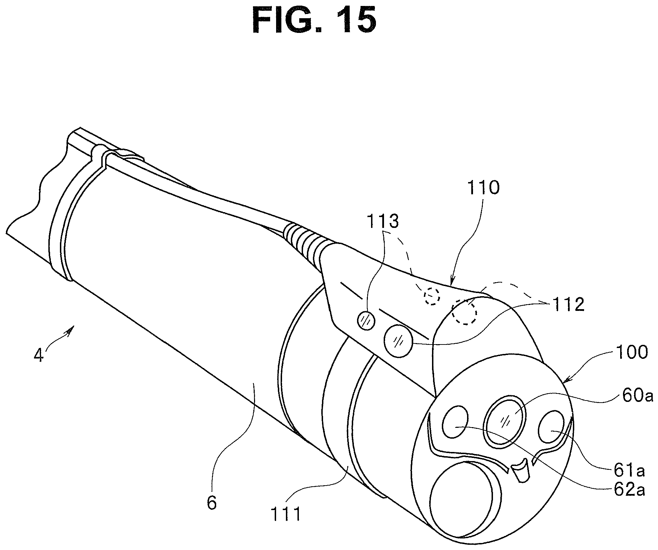

Fig. 15 is a perspective view of the distal end portion 6 of the insertion portion 4 to which a side observation unit is attached. The distal end portion 6 of the insertion portion 4 includes a forward visual field unit 100. A side visual field unit 110 is configured to be detachable from the forward visual field unit 100 by means of a clip portion 111.

The side visual field unit 110 includes two observation windows 112 to acquire images in left and right directions and two illuminating windows 113 to illuminate the left and right directions.

The video processor 32 or the like may be configured to turn on or off the respective illuminating windows 113 of the side visual field unit 110 in accordance with a frame rate of the forward visual field so as to be able to acquire and display an observation image as shown in the aforementioned embodiments.

The present invention is not limited to the aforementioned embodiments, but can be changed, modified or the like in various ways without departing from the spirit and scope of the present invention.

The present application claims priority based on Japanese Patent Application No. 2014-026834, filed on February 14, 2014, the disclosure of which is incorporated in the specification, claims and drawings of the present application by reference.

## Claims

1. An endoscope system comprising:
an insertion portion configured to be inserted into an object;
a first object image acquisition section provided in the insertion portion and configured to acquire a first object image from a first region of the object;
a second object image acquisition section provided in the insertion portion and configured to acquire a second object image from a second region of the object which is at least partially different from the first region; and
an image signal generation section configured to generate an image signal when generating a second image signal from the second object image by performing image processing as well as generating a first image signal from the first object image so that a width of an image region increases as a distance from a region adjacent to the first image signal increases in a positional relationship with the first image signal.

2. The endoscope system according to claim 1, further comprising a display section configured to display an image signal outputted from the image signal generation section.

3. The endoscope system according to claim 2, wherein the image signal generation section causes the display section to display the first object image and the second object image so as to be arranged adjacent to each other.

4. The endoscope system according to claim 3, wherein the display section comprises at least two or more monitors, and
the image signal generation section outputs the first image signal and the second image signal to the monitors by removing an overlapping region between the image signals.

5. The endoscope system according to claim 3, wherein the image signal generation section causes the display section to display the first object image arranged at a center and the second object image arranged in regions including at least both sides of the first object image.

6. The endoscope system according to claim 2, wherein the image signal generation section causes the display section to display the first object image and the second object image on one screen.

7. The endoscope system according to claim 1,
wherein the first object image is the object image of the first region including an insertion portion forward direction substantially parallel to a longitudinal direction of the insertion portion,
the second object image is the object image of the second region including an insertion portion side direction in a direction crossing the longitudinal direction of the insertion portion,
the first object image acquisition section is a forward direction image acquisition section configured to acquire the object image of the first region, and
the second object image acquisition section is a side direction image acquisition section configured to acquire the object image of the second region.

8. The endoscope system according to claim 1, wherein the image signal generation section performs image processing on the second image signal in a first switchover mode configured to cancel a mode in which the width of the image region increases as the distance from the region adjacent to the first image signal increases for only a region of interest which is a specified predetermined region in the second object image.

9. The endoscope system according to claim 8, wherein the image signal generation section performs image processing on the second image signal in a second switchover mode in which only the region of interest in the second object image is enlarged and displayed.

10. The endoscope system according to claim 1,
wherein the image signal generation section performs image processing on the second image signal in a third switchover mode configured to cancel a mode in which the width of the image region increases as the distance from the region adjacent to the first image signal increases and display an enlarged image for only the entire second object image where a region of interest which is a specified predetermined region in the second object image exists.

11. The endoscope system according to claim 1, wherein the image signal generation section performs image processing on the second image signal in a fourth switchover mode in which distortion in horizontal and vertical directions of the specified second object image is suppressed.

12. The endoscope system according to claim 11, wherein the image signal generation section further performs image processing on the first object image as well in a fifth switchover mode in which distortion in horizontal and vertical directions of the first object image is suppressed.

13. The endoscope system according to claim 5,
wherein the second object image acquisition section in plurality is arranged in a circumferential direction of the insertion portion at substantially uniform angles,
the display section arranges the first object image at a center and displays the second object image in plurality so as to be arranged in a circumferential direction of the first object image at substantially uniform angles, and
the image signal generation section applies to the respective second image signals, a mode in which the width of the image region increases as the distance from the first image signal increases.

14. The endoscope system according to claim 7,
wherein the first object image acquisition section is disposed at a distal end portion of the insertion portion in the longitudinal direction toward a direction in which the insertion portion is inserted,
the second object image acquisition section is disposed on a side face of the insertion portion toward a circumferential direction of the insertion portion, and
a first image pickup section configured to photoelectrically convert the first object image from the first object image acquisition section and a second image pickup section configured to photoelectrically convert the second object image from the second object image acquisition section are provided separately, and the first image pickup section and the second image pickup section are electrically connected to the image signal generation section.

15. The endoscope system according to claim 7,
wherein the first object image acquisition section is disposed at a distal end portion of the insertion portion in the longitudinal direction toward a direction in which the insertion portion is inserted,
the second object image acquisition section is disposed so as to surround a circumferential direction of the insertion portion, and
the endoscope system comprises an image pickup section disposed so as to photoelectrically convert the first object image from the first object image acquisition section and the second object image from the second object image acquisition section on a same surface, and electrically connected to the image signal generation section.
